(19) **Europäisches Patentamt / European Patent Office / Office européen des brevets**

(11) **EP 4 120 992 B1**

(12) **EUROPEAN PATENT SPECIFICATION**

(45) Date of publication and mention of the grant of the patent:
**22.11.2023 Bulletin 2023/47**

(21) Application number: **21711267.1**

(22) Date of filing: **15.03.2021**

(51) International Patent Classification (IPC):
*A61K 8/25* $^{(2006.01)}$    *A61K 8/66* $^{(2006.01)}$
*A61K 8/73* $^{(2006.01)}$    *A61Q 11/00* $^{(2006.01)}$

(52) Cooperative Patent Classification (CPC):
**A61K 8/25; A61K 8/66; A61K 8/73; A61Q 11/00**

(86) International application number:
**PCT/EP2021/056571**

(87) International publication number:
**WO 2021/185783 (23.09.2021 Gazette 2021/38)**

(54) **COMPOSITION**

ZUSAMMENSETZUNG

COMPOSITION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.03.2020 EP 20163716**

(43) Date of publication of application:
**25.01.2023 Bulletin 2023/04**

(73) Proprietors:
- **Unilever IP Holdings B.V.**
  **3013 AL Rotterdam (NL)**
  Designated Contracting States:
  **AL AT BE BG CH CZ DK EE ES FI FR GR HR HU IS LI LT LU LV MC MK NL NO PL PT RO SE SI SK SM**
- **Unilever Global IP Limited**
  **Wirral, Merseyside CH62 4ZD (GB)**
  Designated Contracting States:
  **CY DE GB IE IT MT RS TR**

(72) Inventors:
- **MARRIOTT, Robert, Edward**
  **Wirral Merseyside CH63 3JW (GB)**
- **RILEY, Robert, George**
  **Wirral Merseyside CH63 3JW (GB)**
- **TORRES, Ophelie, Marie-Pierre, Cecile**
  **Wirral Merseyside CH63 3JW (GB)**

(74) Representative: **Tansley, Sally Elizabeth**
**Unilever Patent Group**
**Bronland 14**
**6708 WH Wageningen (NL)**

(56) References cited:
**EP-A1- 3 192 496          JP-A- 2001 114 659**
**US-A1- 2015 320 654**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The present invention is concerned with compositions comprising enzymes. More particularly, the present invention is concerned with oral care compositions containing enzymes.

### Background of the Invention

Oral care compositions containing enzymes are described in EP3192496, EP3192495 and EP3192565 and EP3192564. However there remains the need to formulate compositions with enhanced enzyme activity.

The present application relates to a composition and the use thereof to provide an acceptable syneresis without a detrimental effect on enzyme activity in toothpaste formulations.

### Description of the Invention

The present invention relates to a toothpaste composition comprising an enzyme, a surfactant comprising a polyethylene glycol ethers of fatty alcohols, a silica abrasive, carrageenan and a natural gum selected from gum karaya, guar gum, xanthan gum, gum arabic, gum tragacanth and mixtures thereof, in which the weight ratio of xanthan gum to carrageenan is from 1:3 to 1:1.

The invention further relates to the use of natural gum and carrageenan at a weight ratio 1:3 to 1:1 to provide an acceptable syneresis without a detrimental effect on enzyme activity in a toothpaste in which the natural gum is selected from gum karaya, guar gum, xanthan gum, gum arabic, gum tragacanth and mixtures thereof.

For the avoidance of doubt, any feature of one aspect of the present invention may be utilised in any other aspect of the invention. Any feature described as 'preferred' should be understood to be particularly preferred in combination with a further preferred feature or features. Herein, any feature of a particular embodiment may be utilized in any other embodiment of the invention. The word 'comprising' is intended to mean 'including' but not necessarily 'consisting of' or 'composed of'. In other words, the listed steps or options need not be exhaustive. The examples given in the description below are intended to clarify the invention but not to limit the invention. All percentages are weight/weight percentages unless otherwise indicated. Except in the operating and comparative examples, or where otherwise explicitly indicated, all numbers in this description indicating amounts of material or conditions of reaction, physical properties or materials and/or use are to be understood as modified by the word 'about'. Numerical ranges expressed in the format 'from x to y' are understood to include x and y, unless specified otherwise. When for a specific feature multiple preferred ranges are described in the format 'from x to y', it is understood that all ranges combining the different endpoints are also contemplated.

All natural materials named in the application are sourced from chemical companies in Europe.

### Detailed Description of the Invention

Compositions according to the invention comprise carrageenan. It is preferable if the composition comprises greater that 50wt% of the total level of carrageenan of λ-carrageenan. Preferably the weight ratio of κ-carrageenan to λ-carrageenan is from 1:3 to 2:3, preferably the weight ratio of i-carrageenan to λ-carrageenan is from 1:6 to 1:9. It is preferable if the carrageenan comprises a mix of λ-carrageenan, κ-carrageenan. i-carrageenan may also be present.

Preferably the level of carrageenan is from 0.1 wt% to 1.5 wt% of the total composition, more preferably from 0.2wt% to 1.0 wt%.

Compositions of the invention comprise a natural gum selected from gum karaya, guar gum, xanthan gum, gum arabic, and gum tragacanth, preferably the compositions of the invention comprise xanthan gum. Preferably the level of natural gum, preferably xanthan gum is from 0.05 wt% to 1wt% of the total composition, more preferably from 0.3wt% to 0.7 wt%.

The application relates to compositions comprising enzymes, preferably the compositions are personal care compositions and more preferably the compositions are oral care compositions. Preferred product forms for compositions of the invention are those which are suitable for brushing and/or rinsing the surfaces of the oral cavity.

The composition of the invention is not intentionally swallowed for purposes of systemic administration of therapeutic agents, but is applied to the oral cavity, used to treat the oral cavity and then expectorated. Typically such a composition is used in conjunction with a cleaning implement such as a toothbrush, usually by applying it to the bristles of the toothbrush and then brushing the accessible surfaces of the oral cavity.

The toothpaste is in the form of an extrudable semi-solid such as a cream, paste or gel (or mixture thereof).

Compositions according to the invention comprise enzymes. Preferably the enzymes are selected from the following groups:

i) a glycoside hydrolase, more preferably an amylase, most preferably amyloglucosidase;
ii) an oxoreductase acting on OH groups of donors, more preferably an oxidase with oxygen as acceptor, most preferably glucose oxidase;
iii) a hemeperoxidase more preferably a haloperoxidase most preferably a lactoperoxidase.

It is preferred if the enzyme is a mixture of 2 of the above, more preferably 3 of the above, most preferably a mixture of amylglucosidase, glucose oxidase and lactoperoxidase.

In the context of the present invention glucose oxidase either alone or part of the enzyme mix is particularly preferred.

Preferably the total level of enzyme is from 0.01 wt% to 2wt% of the total composition. More preferably from 0.05 wt% to 1 wt% of the total composition.

Preferably the total level of glycoside hydrolase in the composition is from 0.05 to 1 wt% of the total composition.

Preferably the total level of oxoreductase acting on OH groups of donors is from 0.05 to 1 wt% of the total composition.

Preferably the total level of hemeperoxidase is from 0.0005 to 0.01 wt% of the total composition.

Preferably the weight ratio of glycoside hydrolase to other enzymes in the composition is greater than 1:1, more preferably greater than 3:2.

Compositions of the invention may also comprise other proteins such as lysozymes, lactoferrins and colostrum. Preferably these other proteins are present at total levels from 0.001 to 0.5 wt% of the total composition, preferably from 0.005 to 0.1 wt% of the total composition.

Composition according to the invention comprise a silica. The preferred silica used in the present invention is a silica with a low refractive index. It may be used as the sole abrasive silica, or in conjunction with a low level of other abrasive silicas, e.g. those according to EP 236070. The low refractive index silicas, used as abrasives in the present invention are preferably silicas with an apparent refractive index (R.I.) in the range from 1.41 to1.47, more preferably from 1.43 to1.45, preferably having a weight mean particle size of from 5 to 15 $\mu$m, a BET (nitrogen) surface area of from 10 to 150 m$^2$/g, more preferably and an oil absorption of about 70 -150 cm$^3$/100 g, but abrasive silicas with a lower apparent refractive index may also be used. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Huber, Sorbosil AC 77 ex Crosfield Chemicals (having an R.I. of approximately 1.440). The amount of these silicas in the composition generally ranges from 5 to 60% by weight of the total composition more preferably from 5 to 20% by weight.

In some embodiments it is preferred if the enzyme is adsorbed/absorbed onto a thickening silica. Preferably the silica has an oil absorbing level of less than 150 cm$^3$/100g, more preferably less than 1 cm$^3$/100g. Particularly preferred silicas are Zeodent 165 and Tixosil 43 as silicas of this type are preferred as they enhance enzyme activity.

The oral care composition will contain a surfactant comprising a polyethylene glycol ethers of fatty alcohols in an amount from 0.2 to 7% by weight of the composition. Particularly preferred are polyethylene glycol ethers having from 20 to 40 ethylene oxide groups per unit. Examples of such suitable surfactant include the group of surfactants known as Steareth surfactants such as Steareth 30. Preferably the level of non-ionic surfactant is from 0.5 to 7 wt% of the total composition, more preferably from 1 to 5 wt% of the total composition.

Although other surfactants may be present it is preferred if they are limited to levels below 0.5 wt% of the total composition preferably less than 0.1 wt% of the total composition.

In preferred product forms, the amount of water and/or polyhydric alcohol will generally be at least 10 percent, preferably at least 30 percent, more preferably at least 50 percent by total weight water and/or polyhydric alcohol based on the total weight of the composition. Preferably the level of water and/or polyhydric alcohol will be less than 90 wt% of the total composition, more preferably less than 85 wt%.

A dentifrice composition according to the invention will usually contain, as the aqueous continuous phase, a mixture of water and polyhydric alcohol in various relative amounts, with the amount of water generally ranging from 10 to 50% by weight (based on the total weight of the dentifrice) and the amount of polyhydric alcohol generally ranging from 20 to 60% by weight (based on the total weight of the dentifrice).

Typical polyhydric alcohols for use in the oral care composition, particularly a dentifrice composition according to the invention include humectants such as glycerol, sorbitol, polyethylene glycol, polypropylene glycol, propylene glycol, xylitol (and other edible polyhydric alcohols), hydrogenated partially hydrolysed polysaccharides and mixtures thereof. Glycerol and sorbitol are preferred, sorbitol being particularly preferred.

The oral care composition, in particular dentifrice compositions comprises abrasive materials. Abrasive materials will generally be present in an amount of from 0.5 to 75%, preferably 3 to 60% by weight based on the total weight of the dentifrice. Suitable abrasive cleaning agents include silica xerogels, hydrogels and aerogels and precipitated particulate silicas; calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof. Silicas are particularly preferred as they can perform a dual function an abrasive and a means of enhancing the activity of the enzyme. Preferably the composition, particularly a toothpaste, comprises a silica based abrasive as described above.

The composition can comprise additional inorganic or a natural or synthetic thickener or gelling agent in proportions of about 0.10 to about 15% by weight of the total composition, depending on the material chosen. Suitable thickeners or gelling agents useful in the practice of the present invention include finely divided silicas, hectorites, calcium carbonate, colloidal magnesium aluminium silicates and mixtures thereof. Preferred are inorganic thickening silicas such as amorphous silicas available from Evonik under the trade designation Zeodent 165.

Compositions of the invention may comprise a metal ion such as tin or zinc. Preferably the sources of zinc ions are zinc chloride, zinc acetate, zinc gluconate, zinc sulphate, zinc fluoride, zinc citrate, zinc lactate, zinc oxide, zinc monoglycerolate, zinc tartrate, zinc pyrophosphate zinc maleate and mixtures thereof.

Compositions of the invention may comprise fluoride sources such as sodium fluoride, stannous fluoride, sodium monofluorophosphate, zinc ammonium fluoride, tin ammonium fluoride, calcium fluoride, cobalt ammonium fluoride and mixtures thereof.

Compositions of the present invention may also contain further optional ingredients customary in the art such as antimicrobial agents, e.g. chlorhexidine, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);

anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
plant-derivable antioxidants such as flavonoid, catechin, polyphenol, and tannin compounds and mixtures thereof;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
amino acids such as arginine
colouring agents;
pH-adjusting agents;
sweetening agents;
pharmaceutically acceptable carriers, e.g. starch, sucrose, water or water/alcohol systems etc.; surfactants, such as anionic, nonionic, cationic and zwitterionic or amphoteric surfactants; humectants such as glycerol, sorbitol, propyleneglycol, xylitol, lactitol etc.;
polymeric compounds which can enhance the delivery of active ingredients such as antimicrobial agents can also be included;
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

The invention will now be illustrated by the following non-limiting Examples:

## Examples

Compositions were prepared according to Table 1 (Examples A-D are reference examples)

EP 4 120 992 B1

**Table 1**

| Material | A | B | C | D | 1 |
|---|---|---|---|---|---|
| Sorbitol | 28.5 | 28.5 | 28.5 | 28.5 | 28.5 |
| Zinc Gluconate | 0.3 | 0.3 | 0.3 | 0.3 | 0.3 |
| Sodium Fluoride | 0.3 | 0.3 | 0.3 | 0.3 | 0.3 |
| Citric Acid | 0.3 | 0.3 | 0.3 | 0.3 | 0.3 |
| Disodium Phosphate | 0.5 | 0.5 | 0.5 | 0.5 | 0.5 |
| Titanium Dioxide | 0.5 | 0.5 | 0.5 | 0.5 | 0.5 |
| Zeodent 113* | 16.0 | 16.0 | 16.0 | 16.0 | 16.0 |
| Tixosil 43** | 4.8 | 4.8 | 4.8 | 4.8 | 4.8 |
| Carrageenan | 0.7 | 0.7 | 0.7 | 0.7 | 0.7 |
| Glycerin | 5.0 | 5.0 | 5.0 | 5.0 | 5.0 |
| Xanthan gum | 1.0 | 0 | 0.8 | 0.2 | 0.4 |
| Steareth 30 | 3.0 | 3.0 | 3.0 | 3.0 | 3.0 |
| Amyloglucosidase | 0.3 | 0.3 | 0.3 | 0.3 | 0.3 |
| Glucose oxidase | 0.14 | 0.14 | 0.14 | 0.14 | 0.14 |
| Water and minors | to100.0 | to100.0 | to100.0 | to100.0 | to100.0 |
| (composition contains 1450ppm F as NaF)<br>* Precipitated silica<br>** Thickening silica | | | | | |

Syneresis of polymer gels is attributed to changes in the physical and chemical properties of a polymer gel over time. For instance, physical changes of the polymer gel are linked to osmotic pressure and elasticity adjustments of the polymer, occurring due to the rearrangement of the polymer chains. Chemical changes can be linked, for instance, to hydrogen bonding or electrostatic interactions. All these physico-chemical changes lead to the strengthening of the polymer matrix through the aggregation of polymer chains forming the gel network, which in turn expels the continuous phase. Therefore, controlling the aggregation process of the polymer chains, over time limits syneresis. A method to measure syneresis and monitor the aggregation process of polymer chains can be conducted by examining the behaviour of a paste undergoing thermal change over short period of times (i.e., two to three hours). Thermally treating a sample increases the rate at which physical and chemical interactions changes over time and hence decreases the experimental time, which in turn increases the experimental throughput. Temperature cycles alter (i.e., break, form or strengthen) the interactions and bonding (i.e., hydrophobic interactions, hydrogen bonding, etc...) of polymer chains. These different interactions result in variations of the structure and network, potentially destabilising the paste. The measurement of the elastic (G') and viscous (G") modulus as well as $\tan\delta$ (= G"/G') via temperature cycles allows the understanding of these physico-chemical changes. For instance, a decrease in G' over an increase in temperature highlights the reversible breakdown of hydrogen bonds resulting in a more viscous and liquid-like sample. As the temperature cools back down new intermolecular interactions (ionic and H-bonding) will occur resulting in a change of the structure. The extent of the disruption of the structure allows to estimate samples that are more prone to undergo syneresis over time.

For comparison purposes, the changes in G' as the temperature increases ($\Delta G'$) will be examined to predict

$$\Delta G' = \frac{G'_{\min(25-75°C)} - G'_{(25°C)}}{G'_{(25°C)}}$$

the extent of syneresis that occurs in a sample: , where $G'_{(25°C)}$ is the elastic modulus of the gel measured at 25 °C and $G'_{min(25-75°C)}$ is the minimum elastic modulus measured during the temperature increase between 25 and 75 °C.

Table 2 summarises the rules for potential samples undergoing syneresis as a function of $\Delta G'$.

5

Table 2. Rules for syneresis to be observed as a function of $\Delta G'$

| Thermal ramp test $\Delta G'$ | Syneresis observation |
|---|---|
| 0.2 < | No softening of the paste - no syneresis observed |
| -0.3 < $\Delta G'$ < 0.2 | Softening of the paste - no obvious sign of syneresis |
| < -0.3 | Clear softening of the clear - significant syneresis |

The Elastic modulus (G'), of toothpastes was measured in the presence or absence of xanthan gum as was the glucose oxidase activity. The results are shown in Table 3.

**Table 3**

| Sample Ref. | [CR] | [XG] | $\Delta G'$ | Glucose oxidase analysis | |
|---|---|---|---|---|---|
| | | | | Activity | St.d. |
| | (%) | (%) | | (rfu) | (±) |
| A | 0.7 | 1 | 0.71 | 268 | 18 |
| B | 0.7 | 0 | -0.68 | 407 | 23 |

Table 2 demonstrates that 0 wt% XG leads to a significant improvement in GOx activity but syneresis increases.

**Table 4.** Changes in the elastic modulus ($\Delta G'$) over temperature increase of toothpaste containing different concentrations of xanthan gum ([XG]) was measured. The activity of glucose oxidase (GOx) and the viscosity of each sample is also reported.

**Table 4**

| Sample Ref. | [CR] | [XG] | [Ti] | [CR:XG] | [CR + XG + Ti] | η | $\Delta G'$ | Glucose oxidase analysis | |
|---|---|---|---|---|---|---|---|---|---|
| | | | | | | | | Activity | Std. |
| | (%) | (%) | (%) | | (%) | Pa s | | (rfu) | (±) |
| A | 0.7 | 1 | 4.8 | 0.70 | 6.5 | 211 | 0.71 | 205 | 20 |
| C | 0.7 | 0.8 | 4.8 | 0.88 | 6.3 | 215 | -0.20 | 233 | 49 |
| 1 | 0.7 | 0.4 | 4.8 | 1.75 | 5.9 | 155 | -0.27 | 268 | 44 |
| D | 0.7 | 0.2 | 4.8 | 3.50 | 5.7 | 132 | -0.64 | 290 | 41 |

The examples demonstrate that the Example of the invention has acceptable syneresis without detrimental effect on enzyme activity.

**Claims**

1. A toothpaste composition comprising an enzyme, a surfactant comprising a polyethylene glycol ethers of fatty alcohols, a silica abrasive, carrageenan and a natural gum selected from gum karaya, guar gum, xanthan gum, gum arabic, gum tragacanth and mixtures thereof, in which the weight ratio of natural gum to carrageenan is from 1:3 to 1:1.

2. A composition according to claim I in which the weight ratio of natural gum to carrageenan is from 1:2 to 1:1.

3. A composition according to any preceding claim in which the natural gum is xanthan gum.

4. A composition according to any preceding claim in which the enzyme is selected from a glycoside hydrolase, an oxoreductase acting on OH groups of donors, a hemeperoxidase or mixtures thereof.

5. A composition according to any preceding claim in which the enzymes are selected from an amylase, an oxidase with oxygen as acceptor, a haloperoxidase or mixtures thereof.

6. A composition according to any preceding claim in which the enzyme is selected from the group consisting of amylglucosidase, glucose oxidase, lactoperoxidase or mixtures thereof.

7. A composition according to any preceding claim in which the enzyme or enzyme mix comprises glucose oxidase.

8. A composition according to any preceding claim in which the composition comprises a mixture of two enzymes described in any previous claims.

9. A composition according to any preceding claim in which the composition comprises a mixture of three enzymes described in any preceding claim.

10. A composition according to any preceding claim that further comprises a thickening silica.

11. A composition according to any preceding claim in which the carrageenan comprises at least 50 wt% of the total level of carrageenan of λ-carrageenan.

12. A composition according to any preceding claim in which the carrageenan comprises λ-carrageenan, κ-carrageenan and i-carrageenan at a weight ratio of 9:4:1 to 6:3:1.

13. A composition according to any preceding claim in which the polyethylene glycol ethers of a fatty alcohol comprises from 20 to 40 ethylene oxide groups per unit.

14. Use of natural gum and carrageenan at a weight ratio 1:3 to 1:1 to provide an acceptable syneresis without a detrimental effect on enzyme activity in a toothpaste, in which the natural gum is selected from gum karaya, guar gum, xanthan gum, gum arabic, gum tragacanth and mixtures thereof.


**Patentansprüche**

1. Zahnpastazusammensetzung, umfassend ein Enzym, ein Tensid, umfassend Polyethylenglycolether von Fettalkoholen, ein Siliziumdioxidschleifmittel, Carrageenan und einen natürlichen Gummi, ausgewählt aus Karayagummi, Guargummi, Xanthangummi, Gummi arabicum, Traganthgummi und Mischungen davon, wobei das Gewichtsverhältnis von natürlichem Gummi zu Carrageenan zwischen 1:3 bis 1:1 liegt.

2. Zusammensetzung nach Anspruch 1, wobei das Gewichtsverhältnis von natürlichem Gummi zu Carrageenan 1:2 bis 1:1 beträgt.

3. Zusammensetzung nach einem vorhergehenden Anspruch, wobei der natürliche Gummi Xanthangummi ist.

4. Zusammensetzung nach einem vorhergehenden Anspruch, wobei das Enzym aus Glycosidhydrolase, einer auf OH-Gruppen von Donatoren wirkenden Oxoreduktase, einer Hämperoxidase oder Mischungen davon ausgewählt ist.

5. Zusammensetzung nach einem der vorhergehenden Ansprüche, bei der die Enzyme unter einer Amylase, einer Oxidase mit Sauerstoff als Akzeptor, einer Haloperoxidase oder Mischungen davon ausgewählt sind.

6. Zusammensetzung nach einem vorhergehenden Anspruch, wobei das Enzym aus der Gruppe, bestehend aus Amylglucosidase, Glucoseoxidase, Lactoperoxidase oder Mischungen davon, ausgewählt ist.

7. Zusammensetzung nach einem vorhergehenden Anspruch, wobei das Enzym oder das Enzymgemisch Glucoseoxidase umfasst.

8. Zusammensetzung nach einem vorhergehenden Anspruch, wobei die Zusammensetzung eine Mischung aus zwei Enzymen, beschrieben in vorhergehenden Ansprüchen, umfasst.

9. Zusammensetzung nach einem vorhergehenden Anspruch, wobei die Zusammensetzung eine Mischung von drei Enzymen, beschrieben in einem vorhergehenden Anspruch, umfasst.

**10.** Zusammensetzung nach einem vorhergehenden Anspruch, die ferner ein verdickendes Siliziumdioxid umfasst.

**11.** Zusammensetzung nach einem vorhergehenden Anspruch, wobei das Carrageenan mindestens 50 Gew.-% der Gesamtmenge des Carrageenans λ-Carrageenan umfasst.

**12.** Zusammensetzung nach einem vorhergehenden Anspruch, wobei das Carrageenan λ-Carrageenan, κ-Carrageenan und i-Carrageenan in einem Gewichtsverhältnis von 9:4:1 bis 6:3:1 umfasst.

**13.** Zusammensetzung nach einem vorhergehenden Anspruch, wobei die Polyethylenglycolether von Fettalkohol 20 bis 40 Ethylenoxidgruppen pro Einheit umfassen.

**14.** Verwendung von natürlichem Gummi und Carrageenan in einem Gewichtsverhältnis von 1:3 bis 1:1 zum Bereitstellen einer akzeptablen Synärese ohne nachteiligen Effekt auf die Enzymaktivität in Zahnpasta, wobei der natürliche Gummi aus Karayagummi, Guargummi, Xanthangummi, Gummi arabicum, Traganthgummi und Mischungen davon ausgewählt ist.


## Revendications

**1.** Composition de dentifrice comprenant une enzyme, un tensioactif comprenant des éthers de polyéthylène glycol d'alcools gras, un abrasif de silice, du carraghénane et une gomme naturelle choisie parmi de la gomme karaya, gomme guar, gomme xanthane, gomme arabique, gomme adragante et des mélanges de celles-ci, dans laquelle le rapport en masse de gomme naturelle à carraghénane est de 1:3 à 1:1.

**2.** Composition selon la revendication 1, dans laquelle le rapport en masse de gomme naturelle à carraghénane est de 1:2 à 1:1.

**3.** Composition selon l'une quelconque des revendications précédentes, dans laquelle la gomme naturelle est la gomme xanthane.

**4.** Composition selon l'une quelconque des revendications précédentes, dans laquelle l'enzyme est choisie parmi une glycoside hydrolase, une oxoréductase agissant sur des groupes OH de donneurs, une peroxydase héminique ou des mélanges de celles-ci.

**5.** Composition selon l'une quelconque des revendications précédentes, dans laquelle les enzymes sont choisies parmi une amylase, une oxydase avec de l'oxygène comme accepteur, une haloperoxydase ou des mélanges de celles-ci.

**6.** Composition selon l'une quelconque des revendications précédentes, dans laquelle l'enzyme est choisie dans le groupe consistant en amylglucosidase, glucose oxydase, lactoperoxydase ou des mélanges de celles-ci.

**7.** Composition selon l'une quelconque des revendications précédentes, dans laquelle l'enzyme ou le mélange d'enzymes comprend de la glucose oxydase.

**8.** Composition selon l'une quelconque des revendications précédentes, dans laquelle la composition comprend un mélange de deux enzymes décrites dans l'une quelconque des revendications précédentes.

**9.** Composition selon l'une quelconque des revendications précédentes, dans laquelle la composition comprend un mélange de trois enzymes décrites dans l'une quelconque des revendications précédentes.

**10.** Composition selon l'une quelconque des revendications précédentes qui comprend de plus une silice épaississante.

**11.** Composition selon l'une quelconque des revendications précédentes, dans laquelle le carraghénane comprend au moins 50 % en masse de la teneur totale de carraghénane de λ-carraghénane.

**12.** Composition selon l'une quelconque des revendications précédentes, dans laquelle le carraghénane comprend du λ-carraghénane, κ-carraghénane et i-carraghénane à un rapport en masse de 9:4:1 à 6:3:1.

**13.** Composition selon l'une quelconque des revendications précédentes, dans laquelle les éthers de polyéthylène glycol d'un alcool gras comprennent de 20 à 40 groupes oxyde d'éthylène par unité.

**14.** Utilisation de gomme naturelle et de carraghénane à un rapport en masse de 1:3 à 1:1 pour fournir une synérèse acceptable sans effet désavantageux sur l'activité d'enzyme dans un dentifrice, dans laquelle la gomme naturelle est choisie parmi la gomme karaya, gomme guar, gomme xanthane, gomme arabique, gomme adragante et des mélanges de celles-ci.

**EP 4 120 992 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3192496 A
- EP 3192495 A
- EP 3192565 A
- EP 3192564 A